(19) Europäisches Patentamt / European Patent Office / Office européen des brevets

(11) **EP 2 326 723 B1**

(12) **EUROPEAN PATENT SPECIFICATION**

(45) Date of publication and mention
of the grant of the patent:
**06.07.2016 Bulletin 2016/27**

(21) Application number: **09791612.6**

(22) Date of filing: **18.08.2009**

(51) Int Cl.:
***C12P 7/06*** (2006.01)

(86) International application number:
**PCT/US2009/054156**

(87) International publication number:
**WO 2010/022045 (25.02.2010 Gazette 2010/08)**

(54) **PROCESSES FOR PRODUCING FERMENTATION PRODUCTS**

VERFAHREN ZUR HERSTELLUNG VON GÄRUNGSPRODUKTEN

PROCÉDÉS DE PRODUCTION DE PRODUITS DE FERMENTATION

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **20.08.2008 US 90402 P**

(43) Date of publication of application:
**01.06.2011 Bulletin 2011/22**

(73) Proprietor: **Novozymes A/S
2880 Bagsvaerd (DK)**

(72) Inventors:
• **SOONG, Chee-leong
Raleigh
NC 27614 (US)**
• **DEINHAMMER, Randy
Wake Forest
NC 27587 (US)**
• **MATTHEWS, John
Louisburg
NC 27549 (US)**

(56) References cited:
**WO-A2-2004/080923     WO-A2-2007/076388
GB-A- 521 849            GB-A- 822 154
US-A1- 2007 264 696**

• **DATABASE WPI Section Ch, Week 200808
Thomson Scientific, London, GB; Class D16, AN
2008-B27700 XP002564004 -& WO 2007/082976
A1 (MERINO FEBRERO VICENTE [ES]) 26 July
2007 (2007-07-26)**
• **DATABASE WPI Week 198417 Thomson
Scientific, London, GB; AN 1984-103755
XP002564005 & JP 59 045876 A (SAKAI T) 14
March 1984 (1984-03-14)**
• **DATABASE EPODOC EUROPEAN PATENT
OFFICE, THE HAGUE, NL; 4 December 1984
(1984-12-04), SUNTORY LTD: "Preparation of
alcohol" XP002564003 & JP 57 198093 A
(SUNTORY LTD) 4 December 1982 (1982-12-04)**
• **DATABASE WPI Week 198248 Thomson
Scientific, London, GB; AN 1982-03716J
XP002564006 & JP 57 174060 A (IZUMI KAKO KK)
26 October 1982 (1982-10-26)**
• **DATABASE WPI Week 199515 Thomson
Scientific, London, GB; AN 1995-109539
XP002564007 & JP 07 031485 A (MITSUBISHI
JUKOGYO KK) 3 February 1995 (1995-02-03)**

**Description**

**FIELD OF THE INVENTION**

The present invention relates to processes for producing fermentation products from starch-containing material.

**BACKGROUND OF THE INVENTION**

Production of fermentation products, such as ethanol, from starch-containing material is well-known in the art. Generally two different kinds of processes are used. The most commonly used process, often referred to as the "conventional process," includes liquefaction of gelatinized starch at high temperature using typically a bacterial alpha-amylase, followed by simultaneously saccharification and fermentation carried out in the presence of a glucoamylase and a fermenting organism. Another well known process, often referred to as a "raw starch hydrolysis" process (RSH) includes simultaneously saccharifying and fermenting granular starch below the initial gelatinization temperature typically in the presence of an acid fungal alpha-amylase, a glucoamylase, and a fermenting organism.

High temperature liquefaction used in the conventional process generates dextrins and soluble sugars which are further saccarified and fermented. However, as a result of the high temperatures used, Maillard condensation reactions occur between carbonyl groups on reducing sugars and amino groups on amino acids and peptides and form Maillard products. The RSH process does not involve high temperature "cooking" like the conventional prosess. However, factors such as moisture levels and pH can also affect Maillard condensation reactions. In either process, the sugars incorporated into the Maillard products are no longer available for fermentation and overall fermentation product yield is decreased.

Maillard products formed during fermentation processes such as those described above can have additional deleterious effects. For example, the amino acids incorporated into the Maillard products results in a decrease in available essential amino acids, such as lysine, in dried distillers grains, a byproduct of the fermentation process used in applications such as animal feed. Maillard reaction products are also known to inhibit the growth of some fermenting organisms such as yeast, and may inhibit enzymes commonly used in fermentation processes such as alpha-amylases and other carbohydrate source generating enzymes.

Many starch-containing materials, such as corn, contain large amounts of sucrose in addition to starch. Invertase and sucrase are enzymes that typically hydrolyze sucrose into glucose and fructose. However, these enzymes are also known to have transglycosylation activity and are capable of synthesizing oligosaccharides under certain conditions. These and other glycosidic enzymes involved in the hydrolysis of oligosaccharides are also subject to the phenomenon of enzymatic reversion reaction, or condensation, whereby free monosaccharides are linked together with a concomitant release of water. This phenomenon is essentially akin to the reversibility of any chemical reaction.

JP 59 045876 A (Sakai T) concerns a high-order polyploid *Saccharomyces diastaticus* F4 yeast having cycloheximide resistance and enhanced productivity of amylase, sucrase, and ethanol. When used in fermentation in an alcohol process the *Saccharomyces diastaticus* F4 yeast produces sucrase during fermentation. There is still a need for improved processes for producing fermentation products, such as ethanol, from starch-containing material. Process improvements that increase fermentation product yield are highly desirable.

**SUMMARY OF THE INVENTION**

In the first aspect the present invention relates to processes for producing a fermentation product from starch-containing material with a dry solid content in the range from 10-55% w/w comprising:

    (a) liquefying starch-containing material in the presence of an alpha-amylase;
    (b) saccharifying the liquefied material obtained in step (a) using a carbohydrate-source generating enzyme;
    (c) fermenting using a fermenting organism;

wherein an invertase is added before or during the liquefaction step and wherein the process steps are carried out in the following consecutive order: (a), (b) and (c).

**BRIEF DESCRIPTION OF THE FIGURES**

    Figure 1 demonstrates the effect of varying amounts of invertase on ethanol yield over time as measured by weight loss.
    Figure 2 demonstrates the effect of varying amounts of invertase on ethanol yield overtime as measured by HPLC.

**DETAILED DESCRIPTION OF THE INVENTION**

The present invention relates to processes for producing fermentation products, especially ethanol, from starch-containing material, wherein the process includes liquefaction, saccharification, and fermentation steps.

The present invention relates to processes for producing a fermentation product from starch-containing material with a dry solid content in the range from 10-55% w/w comprising:

(a) liquefying starch-containing material in the presence of an alpha-amylase;
(b) saccharifying the liquefied material obtained in step (a) using a carbohydrate-source generating enzyme;
(c) fermenting using a fermenting organism;

wherein an invertase is added before or during the liquefaction step and wherein the process steps are carried out in the following consecutive order: (a), (b) and (c).

In one embodiment the invertase may prevent Maillard reaction products from forming before or during the liquefaction step. The invertase may be synthesizing sucrose or other non-reducing sugars or oligosaccharides from the soluble sugars or polysaccharides available in the slurry prior to or during the liquefaction step. The non-reducing sugars such as sucrose are not susceptible to Maillard reactions but are available for saccarification and fermentation.

In another embodiment, the invertase may enhance hydrolysis of sucrose.

The saccharification step (b) and fermentation step (c) may be carried out either sequentially or simultaneously. In one embodiment, the saccarafication and fermentation steps are carried out sequentially. In another embodiment, the saccarification and fermentation steps are carried out simultaneously.

In a particular embodiment, the process of the invention further comprises, prior to the step (a), the steps of:

x) reducing the particle size of the starch-containing material;
y) forming a slurry comprising the starch-containing material and water.

The aqueous slurry may contain from 10-55% w/w dry solids (DS), preferably 25-45 w/w% DS, more preferably 30-40% w/w DS of starch-containing material. The slurry is heated to above the gelatinization temperature and alpha-amylase, preferably bacterial or acid fungal alpha-amylase may be added to initiate liquefaction (thinning). The slurry may in an embodiment be jet-cooked to further gelatinize the slurry before being subjected to alpha-amylase in step (a).

Liquefaction may in an embodiment be carried out as a three-step hot slurry process. The slurry is heated to between 60-95°C, preferably 80-85°C, and alpha-amylase is added to initiate liquefaction (thinning). Then the slurry may be jet-cooked at a temperature between 95-140°C, preferably 105-125°C, for about 1-15 minutes, preferably for about 3-10 minutes, especially around about 5 minutes. The slurry is cooled to 60-95°C and more alpha-amylase is added to finalize hydrolysis (secondary liquefaction). The liquefaction process is usually carried out at pH 4.5-6.5, in particular at a pH from 5 to 6.

Saccharification step (b) may be carried out using conditions well-know in the art. For instance, a full saccharification process may last up to from about 24 to about 72 hours, however, it is common only to do a pre-saccharification of typically 40-90 minutes at a temperature between 30-65°C, typically about 60°C, followed by complete saccharification during fermentation in a simultaneous saccharification and fermentation process (SSF process). Saccharification is typically carried out at temperatures from 20-75°C, preferably from 40-70°C, typically around 60°C, and at a pH between 4 and 5, normally at about pH 4.5.

The most widely used process in fermentation product, especially ethanol, production is the simultaneous saccharification and fermentation (SSF) process, in which there is no holding stage for the saccharification, meaning that fermenting organism, such as yeast, and enzyme(s), may be added together. SSF may typically be carried out at a temperature from 25°C to 40°C, such as from 28°C to 35°C, such as from 30°C to 34°C, preferably around about 32°C. In an embodiment fermentation is ongoing for 6 to 120 hours, and preferably 24 to 96 hours.

Fermentation Medium

"Fermentation media" or "fermentation medium" refers to the environment in which fermentation is carried out and which includes the fermentation substrate, that is, the carbohydrate source that is metabolized by the fermenting organism.

The fermentation medium may comprise nutrients and growth stimulator(s) for the fermenting organism(s). Nutrient and growth stimulators are widely used in the art of fermentation and include nitrogen sources, such as ammonia; urea, vitamins and minerals, or combinations thereof.

Fermenting Organisms

The term "fermenting organism" refers to any organism, including bacterial and fungal organisms, suitable for use in a fermentation process and capable of producing the desired fermentation product. Especially suitable fermenting organisms are able to ferment, i.e., convert, sugars, such as glucose or maltose, directly or indirectly into the desired fermentation product. Examples of fermenting organisms include fungal organisms, such as yeast. Preferred yeast includes strains of *Saccharomyces* spp., in particular, *Saccharomyces cerevisiae.*

In one embodiment the fermenting organism is added to the fermentation medium so that the viable fermenting organism, such as yeast, count per mL of fermentation medium is in the range from $10^5$ to $10^{12}$, preferably from $10^7$ to $10^{10}$, especially about $5 \times 10^7$.

Commercially available yeast includes, e.g., RED STAR™ and ETHANOL RED™ yeast (available from Fermentis/Lesaffre, USA), FALI (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties).

Starch-Containing Materials

Any suitable starch-containing material may be used according to the present invention. The starting material is generally selected based on the desired fermentation product. Examples of starch-containing materials, suitable for use in a process of the invention, include whole grains, corn, wheat, barley, rye, milo, sago, cassava, tapioca, sorghum, rice, peas, beans, or sweet potatoes, or mixtures thereof, or cereals. Contemplated are also waxy and non-waxy types of corn and barley.

The term "granular starch" means raw uncooked starch, i.e., starch in its natural form found in cereal, tubers or grains. Starch is formed within plant cells as tiny granules insoluble in water. When put in cold water, the starch granules may absorb a small amount of the liquid and swell. At temperatures up to 50°C to 75°C the swelling may be reversible. However, with higher temperatures an irreversible swelling called "gelatinization" begins. Granular starch to be processed may be a highly refined starch quality, preferably at least 90%, at least 95%, at least 97% or at least 99.5% pure or it may be a more crude starch-containing materials comprising (e.g., milled) whole grains including non-starch fractions such as germ residues and fibers. The raw material, such as whole grains, may be reduced in particle size, e.g., by milling, in order to open up the structure and allowing for further processing. Two processes are preferred according to the invention: wet and dry milling. In dry milling whole kernels are milled and used. Wet milling gives a good separation of germ and meal (starch granules and protein) and is often applied at locations where the starch hydrolysate is used in production of, e.g., syrups. Both dry and wet milling is well known in the art of starch processing and is equally contemplated for a process of the invention. In an embodiment the particle size is reduced to between 0.05 to 3.0 mm, preferably 0.1-0.5 mm, or so that at least 30%, preferably at least 50%, more preferably at least 70%, even more preferably at least 90% of the starch-containing material fit through a sieve with a 0.05 to 3.0 mm screen, preferably 0.1-0.5 mm screen.

Fermentation Products

The term "fermentation product" means a product produced by a process including a fermentation step using a fermenting organism. Fermentation products contemplated according to the invention include alcohols (e.g., ethanol, methanol, butanol); organic acids (e.g., citric acid, acetic acid, itaconic acid, lactic acid, succinic acid, gluconic acid); ketones (e.g., acetone); amino acids (e.g., glutamic acid); gases (e.g., $H_2$ and $CO_2$); antibiotics (e.g., penicillin and tetracycline); enzymes; vitamins (e.g., riboflavin, $B_{12}$, beta-carotene); and hormones. In a preferred embodiment the fermentation product is ethanol, e.g., fuel ethanol; drinking ethanol, i.e., potable neutral spirits; or industrial ethanol or products used in the consumable alcohol industry (e.g., beer and wine), dairy industry (e.g., fermented dairy products), leather industry and tobacco industry. Preferred beer types comprise ales, stouts, porters, lagers, bitters, malt liquors, happoushu, high-alcohol beer, low-alcohol beer, low-calorie beer or light beer. Preferred fermentation processes used include alcohol fermentation processes. The fermentation product, such as ethanol, obtained according to the invention, may preferably be used as fuel. However, in the case of ethanol it may also be used as potable ethanol.

Recovery

Subsequent to fermentation the fermentation product may be separated from the fermentation medium. The slurry may be distilled to extract the desired fermentation product or the desired fermentation product may be extracted from the fermentation medium by micro or membrane filtration techniques. Alternatively the fermentation product may be recovered by stripping. Methods for recovery are well known in the art. In one embodiment, the fermentation product, such as ethanol, may optionally be recovered after fermentation by distillation.

## ENZYMES

Even if not specifically mentioned in context of a process of the invention, it is to be understood that enzymes are used in an effective amount.

### Invertase/Sucrase

Invertase is the common name for sucrase. As used herein, "invertase" and "sucrase" have the same meaning. The official name for invertase is beta-fructofuranosidase (E.C. 3.2.1.26) and the reaction catalyzed by this enzyme is the hydrolysis of the terminal nonreducing beta-fructofuranoside residues in beta-fructofuranosides. Invertase is also known to have have transglycosylation activity and is capable of synthesizing oligosaccharides under certain conditions.

The invertase enzyme may be from any source, including bacterial, fungal or plant origin. The invertase is added to the slurry prior to or during liquefaction, in an effective amount. In one embodiment, the invertase is added in the amount of 0.001 to 1.00 mg/g DS. In another embodiment, the invertase is added in the amount of 0.01 to 0.09 mg/g DS, alternatively in the amount of 0.05 to 0.80 mg/g DS, alternatively in the amount of 0.05 to 0.40 mg/g DS, or alternatively in the amount of 0.05 to 0.20 mg/g DS or 0.10 to 0.20 mg/g DS.

Commercial compositions comprising invertase include invertase from baker's yeast and *Candida utilis* (Sigma-Aldrich, St. Louis, MO) invertase from yeast (USB Corporation, Cleveland, OH) Maxinvert® (Centerchem, Inc. Norwalk, CT), Invertase (Advanced Enzyme Technologies, Ltd., Thane, India), and Invertase® (Novozymes, A/S).

### Alpha-Amylase

According to the invention any alpha-amylase may be used, such as of fungal, bacterial or plant origin. In a preferred embodiment the alpha-amylase is an acid alpha-amylase, e.g., acid fungal alpha-amylase or acid bacterial alpha-amylase. The term "acid alpha-amylase" means an alpha-amylase (E.C. 3.2.1.1) which added in an effective amount has activity optimum at a pH in the range of 3 to 7, preferably from 3.5 to 6, or more preferably from 4-5.

Bacterial Alpha-Amylase

According to the invention a bacterial alpha-amylase is preferably derived from the genus *Bacillus.*

In a preferred embodiment the *Bacillus* alpha-amylase is derived from a strain of *Bacillus licheniformis, Bacillus amyloliquefaciens, Bacillus subtilis* or *Bacillus stearothermophilus,* but may also be derived from other *Bacillus* sp. Specific examples of contemplated alpha-amylases include the *Bacillus licheniformis* alpha-amylase shown in SEQ ID NO: 4 in WO 99/19467, the *Bacillus amyloliquefaciens* alpha-amylase SEQ ID NO: 5 in WO 99/19467 and the *Bacillus stearothermophilus* alpha-amylase shown in SEQ ID NO: 3 in WO 99/19467 . In an embodiment the alpha-amylase may be an enzyme having a degree of identity of at least 60%, preferably at least 70%, more preferred at least 80%, even more preferred at least 90%, such as at least 95%, at least 96%, at least 97%, at least 98% or at least 99% to any of the sequences shown in SEQ ID NOS: 1, 2 or 3, respectively, in WO 99/19467.

The *Bacillus* alpha-amylase may also be a variant and/or hybrid, especially one described in any of WO 96/23873, WO 96/23874, WO 97/41213, WO 99/19467, WO 00/60059, and WO 02/10355 . Specifically contemplated alpha-amylase variants are disclosed in US patent nos. 6,093,562, 6,297,038 or US patent no. 6,187,576 and include *Bacillus stearothermophilus* alpha-amylase (BSG alpha-amylase) variants having a deletion of one or two amino acid in positions R179 to G182, preferably a double deletion disclosed in WO 1996/023873 - see e.g., page 20, lines 1-10 , preferably corresponding to delta(181-182) compared to the wild-type BSG alpha-amylase amino acid sequence set forth in SEQ ID NO:3 disclosed in WO 99/19467 or deletion of amino acids R179 and G180 using SEQ ID NO:3 in WO 99/19467 for numbering. Even more preferred are *Bacillus* alpha-amylases, especially *Bacillus stearothermophilus* alpha-amylase, which have a double deletion corresponding to delta(181-182) and further comprise a N193F substitution (also denoted I181* + G182* + N193F) compared to the wild-type BSG alpha-amylase amino acid sequence set forth in SEQ ID NO:3 disclosed in WO 99/19467.

Bacterial Hybrid Alpha-Amylase

A hybrid alpha-amylase specifically contemplated comprises 445 C-terminal amino acid residues of the *Bacillus licheniformis* alpha-amylase (shown in SEQ ID NO: 4 of WO 99/19467) and the 37 N-terminal amino acid residues of the alpha-amylase derived from *Bacillus amyloliquefaciens* (shown in SEQ ID NO: 5 of WO 99/19467), with one or more, especially all, of the following substitution:

G48A+T49I+G107A+H156Y+A181T+N190F+I201F+A209V+Q264S (using the *Bacillus licheniformis* numbering in SEQ ID NO: 4 of WO 99/19467). Also preferred are variants having one or more of the following mutations (or corresponding mutations in other Bacillus alpha-amylase backbones): H154Y, A181T, N190F, A209V and Q264S and/or deletion of two residues between positions 176 and 179, preferably deletion of E178 and G179 (using the SEQ ID NO: 5 numbering of WO 99/19467).

In an embodiment the bacterial alpha-amylase and bacterial hybrid alpha amylase is dosed in an amount of 0.0005-5 KNU per g DS, preferably 0.001-1 KNU per g DS, such as around 0.050 KNU per g DS.

Fungal Alpha-Amylase

Fungal alpha-amylases include alpha-amylases derived from a strain of the genus *Aspergillus,* such as, *Aspergillus oryzae, Aspergillus niger* and *Aspergillis kawachii* alpha-amylases.

A preferred acidic fungal alpha-amylase is a Fungamyl-like alpha-amylase which is derived from a strain of *Aspergillus oryzae.* According to the present invention, the term "Fungamyl-like alpha-amylase" indicates an alpha-amylase which exhibits a high identity, i.e. at least 70%, at least 75%, at least 80%, at least 85%, at least 90%, at least 95%, at least 96%, at least 97%, at least 98%, at least 99% or even 100% identity to the mature part of the amino acid sequence shown in SEQ ID NO: 10 in WO 96/23874.

Another preferred acid alpha-amylase is derived from a strain *Aspergillus niger.* In a preferred embodiment the acid fungal alpha-amylase is the one from *Aspergillus niger* disclosed as "AMYA_ASPNG" in the Swiss-prot/TeEMBL database under the primary accession no. P56271 and described in WO 89/01969 (Example 3). A commercially available acid fungal alpha-amylase derived from *Aspergillus niger is* SP288 (available from Novozymes A/S, Denmark).

Other contemplated wild-type alpha-amylases include those derived from a strain of the genera *Rhizomucor* and *Meripilus,* preferably a strain of *Rhizomucor pusillus* (WO 2004/055178) or *Meripilus giganteus.*

In a preferred embodiment the alpha-amylase is derived from *Aspergillus kawachii* and disclosed by Kaneko et al. J. Ferment. Bioeng 81:292-298(1996) "Molecular-cloning and determination of the nucleotide-sequence of a gene encoding an acid-stable alpha-amylase from *Aspergillus kawachi*"; and further as EMBL: #AB008370.

The fungal alpha-amylase may also be a wild-type enzyme comprising a starch-binding domain (SBD) and an alpha-amylase catalytic domain (i.e., none-hybrid), or a variant thereof. In an embodiment the wild-type alpha-amylase is derived from a strain of *Aspergillus kawachii.*

Fungal Hybrid Alpha-Amylase

In a preferred embodiment the fungal acid alpha-amylase is a hybrid alpha- amylase. Preferred examples of fungal hybrid alpha-amylases include the ones disclosed in WO 2005/003311 or U.S. Patent Publication no. 2005/0054071 (Novozymes) or US patent application no. 60/638,614 (Novozymes). A hybrid alpha-amylase may comprise an alpha-amylase catalytic domain (CD) and a carbohydrate-binding domain/module (CBM), such as a starch binding domain, and optional a linker.

Specific examples of contemplated hybrid alpha-amylases include those disclosed in Table 1 to 5 of the examples in US patent application no. 60/638,614, including Fungamyl variant with catalytic domain JA118 and *Athelia rolfsii* SBD (SEQ ID NO:100 in US 60/638,614), *Rhizomucor pusillus* alpha-amylase with *Athelia rolfsii* AMG linker and SBD (SEQ ID NO:101 in US 60/638,614), *Rhizomucor pusillus* alpha-amylase with *Aspergillus niger* glucoamylase linker and SBD (which is disclosed in Table 5 as a combination of amino acid sequences SEQ ID NO:20, SEQ ID NO:72 and SEQ ID NO:96 in US application no. 11/316,535) or as V039 in Table 5 in WO 2006/069290, and *Meripilus giganteus* alpha-amylase with *Athelia rolfsii* glucoamylase linker and SBD (SEQ ID NO:102 in US 60/638,614). Other specifically contemplated hybrid alpha-amylases are any of the ones listed in Tables 3, 4, 5, and 6 in Example 4 in US application no. 11/316,535 and WO 2006/069290.

Other specific examples of contemplated hybrid alpha-amylases include those disclosed in U.S. Patent Publication no. 2005/0054071, including those disclosed in Table 3 on page 15, such as *Aspergillus niger* alpha-amylase with *Aspergillus kawachii* linker and starch binding domain.

Contemplated are also alpha-amylases which exhibit a high identity to any of above mention alpha-amylases, i.e., at least 70%, at least 75%, at least 80%, at least 85%, at least 90%, at least 95%, at least 96%, at least 97%, at least 98%, at least 99% or even 100% identity to the mature enzyme sequences.

When used according to the present invention, an acid alpha-amylases may be added in an amount of 0.001 to 10 AFAU/g DS, preferably from 0.01 to 5 AFAU/g DS, especially 0.3 to 2 AFAU/g DS. Alternatively, an alpha-amylase may according to the present invention be added in the amout of 0.001 to 1 FAU-F/g DS, preferably 0.01 to 1 FAU-F/g DS.

Commercial Alpha-Amylase Products

Preferred commercial compositions comprising alpha-amylase include MYCOLASE™ from DSM (Gist Brocades), BAN™, TERMAMYL™ SC, FUNGAMYL™, LIQUOZYME™ X, LIQUOZYME™ SC and SAN™ SUPER, SAN™ EXTRA L (Novozymes A/S) and CLARASE™ L-40,000, DEX-LO™, SPEZYME® FRED-L, SPEZYME® HPA, SPEZYME® ALPHA, SPEZYME® XTRA, SPEZYME® AA, SPEZYME® DELTA AA , and GC358 (Genencor Int.), FUELZYME™-LF (Verenium Inc), and the acid fungal alpha-amylase sold under the trade name SP288 (available from Novozymes A/S, Denmark).

**Carbohydrate-Source Generating Enzyme**

The term "carbohydrate-source generating enzyme" includes glucoamylase (being glucose generators), beta-amylase and maltogenic amylase (being maltose generators) and also pullulanase and alpha-glucosidase. A carbohydrate-source generating enzyme is capable of producing a carbohydrate that can be used as an energy-source by the fermenting organism(s) in question, for instance, when used in a process of the invention for producing a fermentation product, such as ethanol. The generated carbohydrate may be converted directly or indirectly to the desired fermentation product, preferably ethanol. According to the invention a mixture of carbohydrate-source generating enzymes may be used. Especially contemplated blends are mixtures comprising at least a glucoamylase and an alpha-amylase, especially an acid amylase, even more preferred an acid fungal alpha-amylase. The ratio between glucoamylase activity (AGU) and fungal alpha-amylase activity (FAU-F) (the ratio of AGU per FAU-F) may in an embodiment of the invention be between 0.1 and 100 AGU/FAU-F, in particular between 2 and 50 AGU/FAU-F, such as in the range from 10-40 AGU/FAU-F.

In a conventional starch-to-ethanol process (i.e., including a liquefaction step (a)) the ratio may preferably be as defined in EP 140,410-B1, especially when saccharification in step (b) and fermentation in step (c) are carried out simultaneously.

Glucoamylase

A glucoamylase used according to the invention may be derived from any suitable source, e.g., derived from a microorganism or a plant. Preferred glucoamylases are of fungal or bacterial origin, selected from the group consisting of *Aspergillus* glucoamylases, in particular *Aspergillus niger* G1 or G2 glucoamylase (Boel et al. (1984), EMBO J. 3 (5), p. 1097-1102), or variants thereof, such as those disclosed in WO 92/00381, WO 00/04136 and WO 01/04273 (from Novozymes, Denmark); the *A. awamori* glucoamylase disclosed in WO 84/02921, *Aspergillus oryzae* glucoamylase (Agric. Biol. Chem. (1991), 55 (4), p. 941-949), or variants or fragments thereof. Other *Aspergillus* glucoamylase variants include variants with enhanced thermal stability: G137A and G139A (Chen et al. (1996), Prot. Eng. 9, 499-505); D257E and D293E/Q (Chen et al. (1995), Prot. Eng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Eng. 10, 1199-1204.

Other glucoamylases include *Athelia rolfsii* (previously denoted *Corticium rolfsii)* glucoamylase (see US patent no. 4,727,026 and (Nagasaka et al. (1998) "Purification and properties of the raw-starch-degrading glucoamylases from Corticium rolfsii, Appl Microbiol Biotechnol 50:323-330), *Talaromyces* glucoamylases, in particular derived from *Talaromyces emersonii* (WO 99/28448), *Talaromyces leycettanus* (US patent no. Re. 32,153), *Talaromyces duponti, Talaromyces thermophilus* (US patent no. 4,587,215).

Bacterial glucoamylases contemplated include glucoamylases from the genus *Clostridium,* in particular C. *thermoamylolyticum* (EP 135,138), and C. *thermohydrosulfuricum* (WO 86/01831) and *Trametes cingulata, Pachykytospora papyracea;* and *Leucopaxillus giganteus* all disclosed in WO 2006/069289; or *Peniophora rufomarginata* disclosed in WO2007/124285; or a mixture thereof. Also hybrid glucoamylase are contemplated according to the invention. Examples the hybrid glucoamylases disclosed in WO 2005/045018. Specific examples include the hybrid glucoamylase disclosed in Table 1 and 4 of Example 1.

Contemplated are also glucoamylases which exhibit a high identity to any of above mention glucoamylases, i.e., at least 70%, at least 75%, at least 80%, at least 85%, at least 90%, at least 95%, at least 96%, at least 97%, at least 98%, at least 99% or even 100% identity to the mature enzymes sequences mentioned above.

Commercially available compositions comprising glucoamylase include AMG 200L; AMG 300 L; SAN™ SUPER, SAN™ EXTRA L, SPIRIZYME™ PLUS, SPIRIZYME™ FUEL, SPIRIZYME™ B4U, SPIRIZYME™ ULTRA and AMG™ E (from Novozymes A/S); OPTIDEX™ 300, GC480, GC147 (from Genencor Int.); AMIGASE™ and AMIGASE™ PLUS (from DSM); G-ZYME® G900, G-ZYME®, G-ZYME® 480 ETHANOL, DISTILLASE® L-400, DISTILLASE® L-500, DISTILLASE® VHP, and G990 ZR (from Genencor Int.).

Glucoamylases may in an embodiment be added in an amount of 0.0001-20 AGU/g DS, preferably 0.001-10 AGU/g DS, especially between 0.01-5 AGU/g DS, such as 0.1-2 AGU/g DS.

Beta-amylase

A beta-amylase (E.C 3.2.1.2) is the name traditionally given to exo-acting maltogenic amylases, which catalyze the hydrolysis of 1,4-alpha-glucosidic linkages in amylose, amylopectin and related glucose polymers. Maltose units are successively removed from the non-reducing chain ends in a step-wise manner until the molecule is degraded or, in the case of amylopectin, until a branch point is reached. The maltose released has the beta anomeric configuration, hence the name beta-amylase.

Beta-amylases have been isolated from various plants and microorganisms (W.M. Fogarty and C.T. Kelly, Progress in Industrial Microbiology, vol. 15, pp. 112-115, 1979). These beta-amylases are characterized by having optimum temperatures in the range from 40°C to 65°C and optimum pH in the range from 4.5 to 7. A commercially available beta-amylase from barley is NOVOZYM™ WBA from Novozymes A/S, Denmark and SPEZYME™ BBA 1500 from Genencor Int., USA.

Maltogenic Amylase

The amylase may also be a maltogenic alpha-amylase. A "maltogenic alpha-amylase" (glucan 1,4-alpha-maltohydrolase, E.C. 3.2.1.133) is able to hydrolyze amylose and amylopectin to maltose in the alpha-configuration. A maltogenic amylase from *Bacillus stearothermophilus* strain NCIB 11837 is commercially available from Novozymes A/S. Maltogenic alpha-amylases are described in US Patent nos. 4,598,048, 4,604,355 and 6,162,628.

The maltogenic amylase may in a preferred embodiment be added in an amount of 0.05-5 mg total protein/gram DS or 0.05- 5 MANU/g DS.

The carbohydrate-source generating enzyme may be any carbohydrate-source generating enzyme, including the ones listed in the "Carbohydrate-Source Generating Enzymes" section above. In a preferred embodiment the carbohydrate-source generating enzyme is a glucoamylase. In an preferred embodiment the glucoamylase is selected from the group derived from a strain of *Aspergillus,* preferably *Aspergillus niger* or *Aspergillus awamori,* a strain of *Talaromyces,* especially *Talaromyces emersonii;* or a strain of *Athelia,* especially *Athelia rolfsii;* a strain of *Trametes,* preferably *Trametes cingulata;* a strain of the genus *Pachykytospora,* preferably a strain of *Pachykytospora papyracea;* or a strain of the genus *Leucopaxillus,* preferably *Leucopaxillus giganteus;* or a strain of the genus *Peniophora,* preferably a strain of the species *Peniophora rufomarginata;* or a mixture thereof.

The alpha-amylase may be any alpha-amylase, including the ones mentioned in the "Alpha-Amylases" section above. In a preferred embodiment the alpha-amylase is an acid alpha-amylase, especially an acid fungal alpha-amylase. In a preferred embodiment the alpha-amylase is selected from the group of fungal alpha-amylases. In a preferred embodiment the alpha-amylase is derived from the genus *Aspergillus,* especially a strain of *A. niger, A. oryzae, A. awamori,* or *Aspergillus kawachii,* or of the genus *Rhizomucor,* preferably a strain the *Rhizomucor pusillus,* or the genus *Meripilus,* preferably a strain of *Meripilus giganteus.*

The ratio between glucoamylase activity (AGU) and fungal alpha-amylase activity (FAU-F) (the ratio of AGU per FAU-F) may in an embodiment of the invention be between 0.1 and 100 AGU/FAU-F, in particular between 2 and 50 AGU/FAU-F, such as in the range from 10-40 AGU/FAU-F. The ratio of acid alpha-amylase to glucoamylase is in the range between 0.3 and 5.0 AFAU/AGU. Above composition of the invention is suitable for use in a process for producing fermentation products, such as ethanol, of the invention.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

**Materials & Methods**

**Materials:**

Glucoamylase T (GA): Glucoamylase derived from *Talaromyces emersonii* and disclosed as SEQ ID NO: 7 in WO 99/28448 and available from Novozymes A7S, Denmark, on request.

Alpha-Amylase A (AAA): *Bacillus stearothermophilus* alpha-amylase variant with the mutations:

I181*+G182*+N193F disclosed in US patent no. 6,187,576 and available on request from Novozymes A/S, Denmark.

Invertase: Invertase derived from baker's yeast 14504-1G, available from Sigma-Aldrich, St. Louis, MO.

Yeast: RED STAR™ available from Red Star/Lesaffre, USA.

**Methods**

**Identity**

The relatedness between two amino acid sequences or between two nucleotide sequences is described by the parameter "identity".

For purposes of the present invention the degree of identity between two amino acid sequences, as well as the degree of identity between two nucleotide sequences, may be determined by the program "align" which is a Needleman-Wunsch alignment (i.e. a global alignment). The program is used for alignment of polypeptide, as well as nucleotide sequences. The default scoring matrix BLOSUM50 is used for polypeptide alignments, and the default identity matrix is used for nucleotide alignments. The penalty for the first residue of a gap is -12 for polypeptides and -16 for nucleotides. The penalties for further residues of a gap are -2 for polypeptides, and -4 for nucleotides.

"Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," Methods in Enzymology 183:63- 98). FASTA protein alignments use the Smith-Waterman algorithm with no limitation on gap size (see "Smith-Waterman algorithm", T. F. Smith and M. S. Waterman (1981) J. Mol. Biol. 147:195-197).

**Glucoamylase activity**

Glucoamylase activity may be measured in Glucoamylase Units (AGU).

**Glucoamylase activity (AGU)**

The Novo Glucoamylase Unit (AGU) is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| AMG incubation: | |
|---|---|
| Substrate: | maltose 23.2 mM |
| Buffer: | acetate 0.1 M |
| pH: | $4.30 \pm 0.05$ |
| Incubation temperature: | $37°C \pm 1$ |
| Reaction time: | 5 minutes |
| Enzyme working range: | 0.5-4.0 AGU/mL |

| Color reaction: | |
|---|---|
| GlucDH: | 430 U/L |
| Mutarotase: | 9 U/L |
| NAD: | 0.21 mM |
| Buffer: | phosphate 0.12 M; 0.15 M NaCl |

(continued)

| Color reaction: | |
|---|---|
| pH: | 7.60 ± 0.05 |
| Incubation temperature: | 37°C ± 1 |
| Reaction time: | 5 minutes |
| Wavelength: | 340 nm |

A folder (EB-SM-0131.02/01) describing this analytical method in more detail is available on request from Novozymes A/S, Denmark, which folder is hereby included by reference.

**Alpha-amylase activity (KNU)**

The alpha-amylase activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.

One Kilo Novo alpha amylase Unit (KNU) is defined as the amount of enzyme which, under standard conditions (i.e., at 37°C +/- 0.05; 0.0003 M $Ca^{2+}$; and pH 5.6) dextrinizes 5260 mg starch dry substance Merck Amylum solubile.

A folder EB-SM-0009.02/01 describing this analytical method in more detail is available upon request to Novozymes A/S, Denmark, which folder is hereby included by reference.

**Acid alpha-amylase activity (AFAU)**

When used according to the present invention the activity of an acid alpha- amylase may be measured in AFAU (Acid Fungal Alpha-amylase Units).

**Acid alpha-amylase activity (AFAU)**

Acid alpha-amylase activity may be measured in AFAU (Acid Fungal Alpha-amylase Units), which are determined relative to an enzyme standard. 1 AFAU is defined as the amount of enzyme which degrades 5.260 mg starch dry matter per hour under the below mentioned standard conditions.

Acid alpha-amylase, an endo-alpha-amylase (1,4-alpha-D-glucan-glucanohydrolase, E.C. 3.2.1.1) hydrolyzes alpha-1,4-glucosidic bonds in the inner regions of the starch molecule to form dextrins and oligosaccharides with different chain lengths. The intensity of color formed with iodine is directly proportional to the concentration of starch. Amylase activity is determined using reverse colorimetry as a reduction in the concentration of starch under the specified analytical conditions.

$$STARCH + IODINE \xrightarrow[40°, pH\ 2,5]{ALPHA - AMYLASE} DEXTRINS + OLIGOSACCHARIDES$$

$\lambda$ = 590 nm

blue/violet t = 23 sec. decoloration

Standard conditions/reaction conditions:

| | |
|---|---|
| Substrate: | Soluble starch, approx. 0.17 g/L |
| Buffer: | Citrate, approx. 0.03 M |
| Iodine (I2): | 0.03 g/L |
| CaCl2: | 1.85 mM |
| pH: | 2.50 ± 0.05 |
| Incubation temperature: | 40°C |

(continued)

| | |
|---|---|
| Reaction time: | 23 seconds |
| Wavelength: | 590 nm |
| Enzyme concentration: | 0.025 AFAU/mL |
| Enzyme working range: | 0.01-0.04 AFAU/mL |

A folder <u>EB-SM-0259.02/01</u> describing this analytical method in more detail is available upon request to Novozymes A/S, Denmark, which folder is hereby included by reference.

**Determination of FAU-F**

FAU-F <u>F</u>ungal <u>A</u>lpha-Amylase <u>U</u>nits (<u>F</u>ungamyl) is measured relative to an enzyme standard of a declared strength.

| Reaction conditions | |
|---|---|
| Temperature | 37°C |
| pH | 7.15 |
| Wavelength | 405 nm |
| Reaction time | 5 min |
| Measuring time | 2 min |

A folder (EB-SM-0216.02) describing this standard method in more detail is available on request from Novozymes A/S, Denmark, which folder is hereby included by reference.

## Examples

**Example 1.** Effect of invertase on fermentation speed and ethanol yield.

| No | Pre-liquefaction / dose (mg/gDS) | | | Liquefaction / dose (NU/gDS) | | SSF / dose (AGU/gDS) | |
|---|---|---|---|---|---|---|---|
| 1 | Invertase | 0.05 | | AAA | 60 | GA | 0.45 |
| 2 | Invertase | 0.1 | | AAA | 60 | GA | 0.45 |
| 3 | Invertase | 0.2 | | AAA | 60 | GA | 0.45 |
| 4 | Invertase | 0.4 | | AAA | 60 | GA | 0.45 |
| 5 | | | | AAA | 60 | GA | 0.45 |

Pre-liquefaction

A 32% (w/v) of corn grain flour with 30% addition of industrial thin-stilage (backset) was suspended in tap water and mixed well. The corn slurry was pH adjusted to pH 6.0. Appropriate amount of invertase, as shown above, was added into the corn slurry and mixed thoroughly then incubated in water bath at 50°C for 2 hours.

Liquefaction

After pre-liquefaction, appropriate amount of liquefaction enzyme (Alpha-Amylase A) was added and the corn slurry mixture was subjected to cooking at 85°C for 2 hours. During liquefaction, the corn slurry was mixed periodically making sure the corn mash blended and cooked properly.

Simultaneous saccharification and fermentation (SSF)

The corn mash was cool to room temperature and subjected to SSF as described below with yeast dosing and determination of ethanol yield by weight loss and HPLC analysis.

Yeast Rehydration

5.5 g of RedStar™ yeast was rehydrated in 100ml distilled water and incubated at 32°C for 30 minutes prior to the beginning of fermentation. Approximately 50 million cells/g DS of yeast were added to each fermentation.

Weight loss method for ethanol yield determination

Urea and Penicillin were added to a final concentration of 0.5ppm and 3mg/L respectively. Small-scale (~4g) fermentations were carried out in 15ml polypropylene tubes with five replicates for each experimental condition. The tubes were prepared by drilling a 1/32 inch (1.5 mm) hole and the empty tubes were then weighed before liquefied corn mash was added. The tubes were weighed again after mash was added to determine the exact weight of mash in each tube. This weight was used to calculate the enzyme dosage necessary as follows:

$$Enz.\ dose\ (ml) = \frac{Final\ enz.\ dose\ (AGU/g\ DS) \times Mash\ weight\ (g) \times Solid\ content\ (\%DS/100)}{(Conc.\ enzyme\ \ AGU/ml)}$$

Enzyme was added according to dosage described in table above and 100μl of rehydrated yeast were added to each tube to begin fermentation. Fermentation progress was followed by weighing the tubes over time for approximately 54 hours. Tubes were vortexed briefly before each weighing. Weight loss values were converted to ethanol yield (g ethanol/g DS) by the following formula:

$$g\ ethanol / g\ DS = \frac{g\ CO_2\ weight\ loss \times \dfrac{1\ mol\ CO_2}{44.0098\ g\ CO_2} \times \dfrac{1\ mol\ ethanol}{1\ mol\ CO_2} \times \dfrac{46.094\ g\ ethanol}{1\ mol\ ethanol}}{g\ corn\ in\ tube\ \ \times\ \ \%DS\ of\ corn}$$

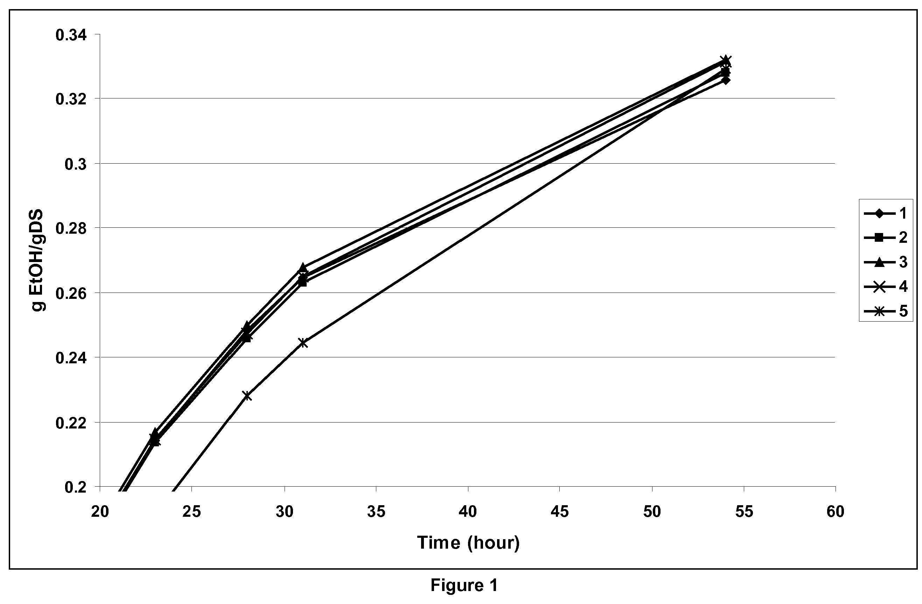

Results are summarized in Figure 1.

HPLC analysis

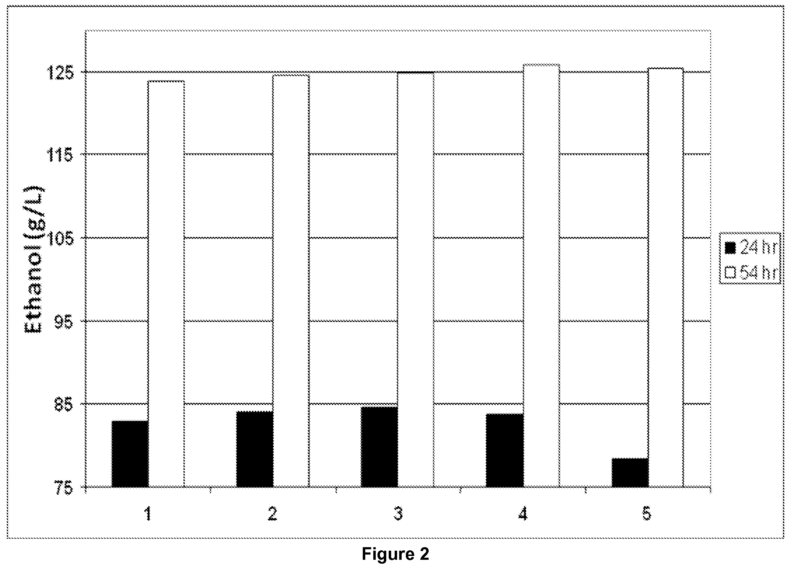

After 24 and 54 hours of fermentation, two replicates respectively from each treatment group were sacrificed for HPLC analysis of remaining sugar and ethanol concentration. The reactions were stopped by adding 50ul 40% $H_2SO_4$ to each tube and mixed well. The tubes were centrifuged at 3000 rpm for 15 minutes to clear the supernatant, and then 1 ml of cleared supernatant was passed through a 0.45μM filter and placed in HPLC vials. The vials were kept at 4°C until analysis. Results are summarized in Figure 2.

**Claims**

1. A process for producing a fermentation product from starch-containing material with a dry solid content in the range from 10-55% w/w comprising:

   (a) liquefying starch-containing material in the presence of an alpha-amylase;
   (b) saccharifying the liquefied material obtained in step (a) using a carbohydrate-source generating enzyme;
   (c) fermenting using a fermenting organism;

   wherein an invertase is added before or during the liquefaction step and wherein the process steps are carried out in the following consecutive order: (a), (b) and (c).

2. The process of claim 1, wherein the invertase is derived from yeast.

3. The process of claims 1 or 2, wherein the invertase is added in the amount of 0.001 to 1.00 mg/g DS.

4. The process of any of claims 1-3, wherein the starch-containing material is derived from corn, wheat, barley, rye, milo, sago, cassava, manioc, tapioca, sorghum, rice or potatoes.

5. The process of any of claims 1-4, wherein the saccharification step (b) and fermentation step (c) are carried out simultaneously.

6. The process of any of claims 1-5, wherein the carbohydrate-source generating enzyme is selected from the group consisting of glucoamylase, alpha-glucosidase, maltogenic amylase, pullulanase, and beta-amylase.

7. The process of any of claims 1-6, wherein the fermentation product is recovered after fermentation.

8. The process of any of claims 1-7, wherein the fermentation product is fuel ethanol, potable ethanol, or industrial ethanol.

9. The process of any of claims 1-8, wherein the fermenting organism is yeast.

10. The process of any of claims 1-9, further comprising, prior to the step (a), the steps of:

> x) reducing the particle size of the starch-containing material;
> y) forming a slurry comprising the starch-containing material and water.


**Patentansprüche**

1. Verfahren zum Herstellen eines Fermentationsprodukts aus stärkehaltigem Material mit einem Trockenmassegehalt im Bereich von 10-55% Gew./Gew., umfassend:

> (a) Verflüssigen von stärkehaltigem Material in der Gegenwart einer alpha-Amylase;
> (b) Verzuckern des in Schritt (a) erhaltenen verflüssigten Materials unter Verwendung eines eine Kohlenhydratquelle erzeugenden Enzyms;
> (c) Fermentieren unter Verwendung eines fermentierenden Organismus;

wobei eine Invertase vor oder nach dem Verflüssigungsschritt zugegeben wird, und wobei die Verfahrensschritte in der folgenden fortlaufenden Reihenfolge ausgeführt werden: (a), (b) und (c).

2. Verfahren nach Anspruch 1, wobei die Invertase aus Hefe abgeleitet ist.

3. Verfahren nach Ansprüchen 1 oder 2, wobei die Invertase in der Menge von 0,001 bis 1,00 mg/g DS zugegeben wird.

4. Verfahren nach einem beliebigen der Ansprüche 1-3, wobei das stärkehaltige Material aus Mais, Weizen, Gerste, Roggen, Mohrenhirse, Sago, Kassava, Maniok, Tapioka, Sorghum, Reis oder Kartoffeln abgeleitet ist.

5. Verfahren nach einem beliebigen der Ansprüche 1-4, wobei der Verzuckerungsschritt (b) und Fermentationsschritt (c) gleichzeitig ausgeführt werden.

6. Verfahren nach einem beliebigen der Ansprüche 1-5, wobei das eine Kohlenhydratquelle erzeugende Enzym ausgewählt ist aus der Gruppe bestehend aus Glucoamylase, alpha-Glucosidase, maltogener Amylase, Pullulanase und beta-Amylase.

7. Verfahren nach einem beliebigen der Ansprüche 1-6, wobei das Fermentationsprodukt nach der Fermentation gewonnen wird.

8. Verfahren nach einem beliebigen der Ansprüche 1-7, wobei das Fermentationsprodukt Treibstoffethanol, Trinkethanol oder Industrieethanol ist.

9. Verfahren nach einem beliebigen der Ansprüche 1-8, wobei der fermentierende Organismus Hefe ist.

10. Verfahren nach einem beliebigen der Ansprüche 1-9, weiterhin umfassend, vor Schritt (a), die Schritte:

x) Verringern der Partikelgröße des stärkehaltigen Materials;

y) Bilden einer das stärkehaltige Material und Wasser umfassenden Aufschlämmung.

**Revendications**

1. Procédé de production d'un produit de fermentation à partir d'une matière contenant de l'amidon avec une teneur en matière sèche située dans la plage allant de 10 à 55 % poids/poids, comprenant :

   (a) la liquéfaction de la matière contenant de l'amidon en présence d'une alpha-amylase ;
   (b) la saccharification de la matière liquéfiée obtenue à l'étape (a) en utilisant une enzyme générant une source d'hydrate de carbone ;
   (c) une fermentation en utilisant un organisme de fermentation ;

   dans lequel une invertase est ajoutée avant ou pendant l'étape de liquéfaction et dans lequel les étapes de procédé sont réalisées dans l'ordre consécutif suivant : (a), (b) et (c).

2. Procédé selon la revendication 1, dans lequel l'invertase est dérivée de la levure.

3. Procédé selon les revendications 1 ou 2, dans lequel l'invertase est ajoutée en la quantité de 0,001 à 1,00 mg/g de matière sèche.

4. Procédé selon l'une quelconque des revendications 1 à 3, dans lequel la matière contenant de l'amidon est dérivée du maïs, du blé, de l'orge, du seigle, du millet, du sagou, de la cassave, du manioc, du tapioca, du sorgho, du riz ou des pommes de terre.

5. Procédé selon l'une quelconque des revendications 1 à 4, dans lequel l'étape de saccharification (b) et l'étape de fermentation (c) sont réalisées simultanément.

6. Procédé selon l'une quelconque des revendications 1 à 5, dans lequel l'enzyme générant la source d'hydrate de carbone est choisie dans le groupe constitué par la glucoamylase, l'alpha-glucosidase, l'amylase maltogénique, la pullulanase et la bêta-amylase.

7. Procédé selon l'une quelconque des revendications 1 à 6, dans lequel le produit de fermentation est récupéré après fermentation.

8. Procédé selon l'une quelconque des revendications 1 à 7, dans lequel le produit de fermentation est le carburant éthanol, l'éthanol buvable ou l'éthanol industriel.

9. Procédé selon l'une quelconque des revendications 1 à 8, dans lequel l'organisme de fermentation est la levure.

10. Procédé selon l'une quelconque des revendications 1 à 9, comprenant en outre, avant l'étape (a), les étapes de :

   x) réduction de la taille des particules de la matière contenant de l'amidon ;
   y) formation d'une suspension comprenant la matière contenant de l'amidon et de l'eau.

**Figure 1**

Figure 2

## REFERENCES CITED IN THE DESCRIPTION

**Patent documents cited in the description**

- JP 59045876 A
- WO 9919467 A
- WO 9623873 A
- WO 9623874 A
- WO 9741213 A
- WO 0060059 A
- WO 0210355 A
- US 6093562 A
- US 6297038 B
- US 6187576 B
- WO 1996023873 A
- WO 8901969 A
- WO 2004055178 A
- WO 2005003311 A
- US 20050054071 A
- US 60638614 B
- US 11316535 B
- WO 2006069290 A
- EP 140410 B1
- WO 9200381 A
- WO 0004136 A
- WO 0104273 A
- WO 8402921 A
- US 4727026 A
- WO 9928448 A
- US RE32153 E
- US 4587215 A
- EP 135138 A
- WO 8601831 A
- WO 2006069289 A
- WO 2007124285 A
- WO 2005045018 A
- US 4598048 A
- US 4604355 A
- US 6162628 A

**Non-patent literature cited in the description**

- **KANEKO et al.** *J. Ferment. Bioeng,* 1996, vol. 81, 292-298
- **BOEL et al.** *EMBO J.,* 1984, vol. 3 (5), 1097-1102
- *Agric. Biol. Chem.,* 1991, vol. 55 (4), 941-949
- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **NAGASAKA et al.** Purification and properties of the raw-starch-degrading glucoamylases from Corticium rolfsii. *Appl Microbiol Biotechnol,* 1998, vol. 50, 323-330
- **W.M. FOGARTY ; C.T. KELLY.** *Progress in Industrial Microbiology,* 1979, vol. 15, 112-115
- **W. R. PEARSON ; D. J. LIPMAN.** Improved Tools for Biological Sequence Analysis. *PNAS,* 1988, vol. 85, 2444-2448
- **W. R. PEARSON.** Rapid and Sensitive Sequence Comparison with FASTP and FASTA. *Methods in Enzymology,* 1990, vol. 183, 63-98
- **T. F. SMITH ; M. S. WATERMAN.** Smith-Waterman algorithm. *J. Mol. Biol.,* 1981, vol. 147, 195-197